# EUROPEAN PATENT SPECIFICATION

(11) **EP 0 512 905 B1**
(45) Date of publication and mention of the grant of the patent: **29.10.1997**
(21) Application number: 92401242.0
(22) Date of filing: 30.04.1992
(51) Int. Cl.: C12P 21/08, C07K 16/00, C12P 21/00, G01N 33/577, C12Q 1/30, A61K 39/395, G01N 33/53

(54) **Heterobifunctional antibodies possessing both catalytic and specific antigen binding properties and methods using them**
Heterobifunktionelle Antikörper mit sowohl katalytischen Eigenschaften als auch spezifischen Antigenbindungseigenschaften und Verfahren zu deren Verwendung
Anticorps hétérobifonctionnels ayant une propriété catalytique et une propriété de liaison spécifique à l'antigène, ainsi que des méthodes utilisant ces anticorps

(30) Priority: 03.05.1991 US 694984; 16.12.1991 US 807130
(43) Date of publication of application: 11.11.1992
(73) Proprietor: PASTEUR SANOFI DIAGNOSTICS, 92430 Marnes La Coquette (FR)
(72) Inventor: Newman, Karel, Eden Prairie, MN 55344 (US); Schmidt, Jane, St. Paul, MN 55105 (US)
(74) Representative: Polus, Camille

(56) References cited:
- EP-A- 0 404 097
- WO-A-91/14769
- METHODS vol. 2, no. 2, April 1991, ORLANDO FL, US pages 97 - 105 M. WHITLOW ET AL. 'Single-chain Fv proteins and their fusion proteins.'
- ESSAYS IN BIOCHEMISTRY vol. 26, 1991, LONDON, GB pages 59 - 75 R. O'KENNEDY ET AL. 'Antibody engineering: an overview.'

## Description

### FIELD OF THE INVENTION

This invention relates to bifunctional antibodies possessing both catalytic activity and antigen binding specificity and to methods of using such antibodies.

### BACKGROUND OF THE INVENTION

Polyclonal antibodies are recognized for their diverse range of specificities. In 1975, Kohler and Milstein published a strategy for immortalizing lymphocytic cell lines (vv. hybridomas) characterized by their ability to produce monoclonal antibodies with singular antigen specificities. Since that development, innumerable hybridomas have been developed with specificities to an equally innumerable number of antigens. Some examples of molecular species recognized by monoclonal antibodies include carbohydrates, amino acid sequences, nucleic acids, conformational features of molecules and inorganic side groups.

Antibodies share certain attributes with a catalytic class of biomolecules known as enzymes. Both antibodies and enzymes are proteinaceous, possess a binding pocket for a corresponding ligand, and have a high degree of specificity for their respective target molecules. The binding pocket of an enzyme provides an arrangement of functional groups to catalyze a specific chemical transformation of the bound ligands.

In 1975, Raso and Stollar, attempted to raise antibodies which specifically bind stable analogs of intermediate which participate in enzymatic and nonenzymatic pyridoxal phosphate catalysis. Although they were successful in obtaining such antibodies, they did not report catalytic activity. (J. Raso and B.D. Stollar, "The Antibody-Enzyme Analogy. Characterization of Antibodies to Phosphopyridoxyltyrosine Derivates," Biochemistry, Vol. 14: 584-591 (1975)).

Tramontano and Lerner, in the mid-eighties, utilized hybridoma technology to select for an antibody that would recognize the transition-state form of a substrate. As described inU.S. Patent 4,900,674, Tramontano et al used a synthetic phosphonate analogue resembling the putative transition-state form of an amide or ester product to develop and identify monoclonal antibodies which recognized the phosphonate substrate analogue, and coincidentally catalyzed the formation or the cleavage of the corresponding carbonylester or amide.

Other catalytic monoclonal antibodies have since been developed. For examples, antibodies with specific catalytic activities have been found which include such activity as lactone cyclization, amide cleavage and acyl group transfer. Each of these catalytic antibodies was developed using an analogue resembling the putative transition-state form of an antigen or hapten to develop and identify the desired antibody. In each case, antibodies were developed that specifically bound to the immunizing hapten or antigen creating an antibody that intrinsically had catalytic capabilities; see Lerner et al., Science 252, p. 659-667 (1991).

Studies show that such antibodies exhibit enzyme characteristics: the catalytic reaction follows Michealis-Menton kinetic principles; the antibodies are not consumed by the reaction; the antibodies display a characteristic rate turnover constant; and the reaction can be inhibited with the original immunogen or analogue thereof.

The term "abzyme" has been coined to refer to an antibody molecule with such catalytic activity as an intrinsic part of the molecule; see Jarda et al., Science 241,. p. 1188-1191 (1988). That term will be used in this specification to describe such antibody molecules.

Another type of antibody molecule has recently been developed through advances in hybridoma technology. U.S. Patent 4,474,893 describes methods of manufacturing antibodies that have binding affinities for two different antigens. Antibodies of this type have been obtained by fusing hybridomas which secrete monoclonal antibodies with binding specificity for one antigen with hydridomas or lymphocytes which manufacture antibodies with binding specificity to a different antigen. The fused cells, referred to as a quadroma or trioma, respectively, produce antibody molecules of which 10%-50% exhibit binding specificity for both antigens; see respectively C. Reading in Hybridomas and Cellular Immortality, p. 235-250, Plenum press (1981) and C. Milstein, A.C. Cuello in Immunology Today, p. 29 (1984).

Antibodies with binding capabilities for two antigens have been referred to as heterobifunctional antibodies. Such antibodies have also been obtained using chemically dissociated antibody halves, followed by reassociation using chemical crosslinking reagents.

Heterobifunctional antibodies were developed by researchers attempting to develop a vehicle for the delivery of therapeutic compounds. Specifically, it was envisioned that an antibody half specific for a target cell coupled with an antibody half specific for a toxin might be a useful vehicle for the delivery of a toxic molecule to a targeted cell. Heterobifunctional antibodies have also been used as reagents in diagnostic assays. For example, Takashi and Fuller, Clin. Chem. 34: 1693 (1988), developed a sensitive assay for human chorionic gonadotropin and Gorog et al, J. Immun. Meth. 123: 131 (1989), developed a homogeneous assay for carcinoembryonal antigen (CEA) using heterobifunctional antibodies having specific binding affinity for both an enzyme and an antigen. The heterobifunctional antibodies were used in place of an antibody-enzyme conjugate.

Methods of cloning and modifying the functional regions of abzymes and antigen-specific binding regions have been described. Iverson et al, Cold Spring Harbor Symp. 56: 273 (1989), described a method for designing an expression vector for expressing abzymes, and a method for expressing the Vl and Vh domains of an antibody.

Despite the advances in the development of new antibodies, further development is desirable. Specifically, studies with conventional enzyme-antibody conjugates used in diagnostic assays indicate that the molecular size of the complex influences the diffusion coefficient in solution, and hence the velocity of associative immunological reaction.

Conventional enzyme-antibody conjugates are produced by introducing reactive groups into both the antibody and enzyme, typically 2-5 groupes per molecule. Because of the number of reactive groups, multiple linkages and aggregate formation are possible. Although conditions are optimized to minimize aggregation, a variable amount of large molecular weight species is also always present. Moreover, the conventional enzyme antibody conjugates are not readily produced in an exact 1:1 ratio of catalytic and binding functionalities, reducing the efficiency of the conjugate performance.

### SUMMARY OF THE INVENTION

The invention relates to antibodies which intrinsically possess both catalytic function and antigen specificity. Each such antibody, which for purposes of this description shall be called a "heterobifunctional catalytic antibody (HCAb), has a first F(ab) portion, the variable domain thereof exhibiting intrinsic catalytic activity to a substrate and a second F(ab) portion, the variable domain thereof being immunologically specific to an antigen different from said substrate. An advantage of such antibodies used as reagents in diagnostic immunoassays or as therapeutic molecules is its low molecular weight. An HCAb would be free of the excess molecular weight of an attached enzyme; further, molecular weight could be further reduced throughout for example the generation of F(ab')2, and in the following by HCAb is meant complete antibodies or active fragments thereof.

For example, a 1:1 conjugate of IgG (160.000 MW) and alkaline phosphatase (140.000 MW) will have a molecular weight of approximately 300.000. An intact HCAb of the invention having the ability to react with a substrate in the same way as alkaline phosphatase and the ability to bind to an antigen would have a molecular weight of approximately 160.000. In a preferred embodiment, the molecular weight of the HCAb may be reduced further by the generation of F(ab')2.

In one embodiment of the invention, a HCAb is directly manufactured, eliminating the multistep process required for production of a standard enzyme-antibody conjugate. As discussed above, conventional enzyme-antibody conjugates are formed using a process that results in a variable amount of aggregate formation. An HCAb, in one embodiment of the invention is produced chemically by forming linkages between sulfhydryls on two different Fabs, one Fab having intrinsic catalytic properties and the other Fab having the ability to specifically bind an antigen. Thus, the conditions greatly favor the formation of a HCAb having a 1:1 ratio.

Yet another embodiment of this invention relates to methods of preparing HCAbs from the hybridization of a catalytic antibody (abzyme) with an antigen specific antibody which is preferably a monoclonal antibody. The products of this hybridization will have a molecular weight similar to parent molecules, yet possess activities of both parent molecules.

This invention involves the application of such HCAbs in sensitive immunoassays for the detection of a particular analyte.

In a preferred embodiment of the invention, HCAbs of the invention are produced having the ability to bind specifically to an analyte being assayed in an immunoassay and intrinsic catalytic capability of acting on a substrate to form a detectable signal. HCAbs of the invention can be substituted for enzyme-antibody conjugates widely used in immunoassays. For example, HCAbs having a catalytic domain with a phosphatase activity and the ability to specifically bind to a tumor marker, a drug such as theophylline, hormones such as TSH, T4, progesterone, vitamins such as B12, and any other analyte for which monoclonal antibodies are available.

This invention also relates to diagnostic kits for assaying for an antigen in samples, comprising a predetermined amount of a HCAb which will bind specifically to the antigen in an amount related to the amount of antigen in the sample and to the substrate to which the antibody exhibits catalytic activity, so that the transformed substrate is detectable or mesurable. The kit also preferably includes a solid support to which the HCAb has been directly or indirectly bound.

In yet another embodiment of the invention, a HCAb is produced that specifically binds to a unique cellular tumor marker and has a catalytic activity toxic so that cell and is used in targeting prodrug activation.

### BRIEF DESCRIPTION OF THE FIGURES

Figure 1 shows an expression system for a preparation of a heterobifunctional catalytic antibody of this invention in a cell host.

Figure 2 shows a Sephacryl® size exclusion column profile of hybrid F(ab')2 (MOPC167 x 9-49 - See Example 2 below).

Figure 3 shows catalysis of p-nitrophenylcarbonylcholine by the heterobifunctional catalytic antibody of the invention when it is concomitantly associated with theophylline immobilized on particles (see example 4 below).

### DETAILED DESCRIPTION OF THE INVENTION

HCAbs of this invention may be obtained through several different methods such as the selective reduction and reannealing of two different antibody molecules or by hybridization of two different hybridomas. Recombinant methods can also be used. The efficiency of recovery will vary with the procedure and the starting materials used.

Non-conventional methods, such as recombinant techniques, wherein the complementary pairing regions within the variable regions of an antibody molecule are designed to produce HCAbs having a desired catalytic activity and having the ability to specifically bind to a desired antigen are likely to improve the efficiency of annealing.

In one embodiment the HCAbs are produced by cell types constructed for the purpose. One cell type is a quadroma and formed by somatic cell fusion of two hybridomas, each parental hybridoma producing a monoclonal antibody, one of the monoclonal antibodies exhibiting catalytic activity to a particular substrate and the other monoclonal being specific for a particular antigen. Another cell type is a trioma and formed by the fusion of a hybridoma and a lymphocyte, each producing antibodies, one type of antibody exhibiting catalytic activity and one antibody being specific to the antigen.

In conventional methods, to obtain a hybridoma which secretes catalytic antibodies that can catalyze a particular reaction, an antigen must first be fabricated that structurally resembles a high-energy intermediate species, anticipated in a chemical reaction. The antigen or hapten is frequently linked to a larger, antigenic carrier molecule. Such antigens or haptens desirably include a transition state analog, such as phosphonate ester in the formation of an amide or ester molecule.

Carrier molecules useful with the invention are well known and are generally proteins. They include tetanus toxoid or keyhole limpet hemocyanin, poly-l-lysine, thyroglobulin, albumins, red blood cells and the like.

Methods of linking carrier molecules to the antigen are well known in the art (see S.H. Wong in Chemistry of protein conjugation and cross-linking - 1991 - CRC Press). Linking groups useful with this invention include m-maleimidobenzoyl-N-hydroxy succinimide ester, glutaraldehyde, N-hydroxysuccinimidyl glutaryl chloride and N-hydroxysuccininimidyl adipoyl chloride.

The antigen-carrier conjugate is used to immunize animals to provide a population of spleen cells producing the desired antibody. Immunization may be accomplished by conventional in vivo methods. Subsequently, splenocytes are fused with cells of one of the myeloma cell lines commercially available, such SP2/O available from ATCC. The supernatants from individual cultures are screened for antibody response using a means such as a 96-well plate to which an antibody to immunoglobulin has been attached. The supernatants are incubated in the plates for a sufficient period for antibody in the supernatants to bind to the plate. Then the substrate which is cleaved by an antibody having the desired catalytic activity is added to the plates and incubated. The plates are periodically examined for signs of catalytic activity. Positive clones are expanded, and evaluated further for evidence of hydrolytic or catalytic activity with the desired substrate.

Separately, a fusion partner is developed, which is either a hybridoma which produces monoclonal antibodies which bind specifically to a desired antigen, or a spleen cell from an animal immunized with the desired antigen.

As mentioned above, HCAbs may be obtained by the formation of a trioma or quadroma. Monoclonal antibody producing hybridomas are produced in the usual manner.

Myeloma cell mutants lacking the enzyme hypoxantine guanine phosphoribosyltransferase (HGPRT) are desirably used. These cells fail to grow in medium containing hypoxanthine, aminopterin and thymidine (HAT). Spleen cells contain HGPRT, but are incapable of continuous division. Thus, only hybridomas with HGPRT (hybridomas fused with spleen cells i.e. triomas) will survive in HAT medium. Such cell lines are termed HAT resistant.

These hybridomas are commonly backselected for HAT sensitivity prior to further fusion.

Backselection is carried out by growing the cells in increasing concentrations of 8-azaguanine (starting concentration 1 microgram/millititer, increasing to 20 micrograms/ milliliter) in complete growth medium to enrich for HGPRT deficient and mutant cells. HAT sensitivity can be similarly produced using bromodeoxyuridine to induce thymidine kinase deficiency.

These back-selected antibody-producing hybridomas can then be fused directly to antibody-secreting spleen cells from an animal immunized with the antigen to form a trioma. Such triomas can then be selected using HAT medium.

To form a quadroma, typically one hybridoma line is maintained with HAT resistance and the other hybridoma line is back-selected for HAT sensitivity and in some instances further modified for resistance to biological inhibitors or cytotoxic drugs. Ouabain has been used most frequently, but emetine, actinomycin and neomycin have also been used. Thus when a HAT-sensitive-ouabain-resistant hybridoma is fused with a HAT-resistant-ouabain-sensitive hybridoma, quadromas will be produced which are both HAT and ouabain resistant and therefore easily identified.

A recombinant expression system may also be used to obtain the HCAbs of this invention by culturing cells transformed by recombinant vectors. Antibody heavy and light chains can be redesigned to afford selective complementarity, as well as linkage of the relevent genes on a single chromosome. A recombinant system would, in fact, be expected to be a more stable system for expression of HCAbs, and would serve as a vehicle for expression of complementarity determining regions from the variable domains of other antibody specificities.

The HCAb molecule including each of the variable domains is a product of the annealing of two types of polypeptide chains (heavy and light) that are encoded by separate genes. As is well-known, the genes that normally encode the synthesis of each chain have a complex organization in which the required nucleic acid base pair sequences appear in a discontinuous linear arrangement. That is, the genetic information required for expression of elements required for construction of the intact heavy or light chain is separated by intervening, non-expressed genetic information. The genetic information required for heavy chain gene expression includes information for the promotor sequence, separate leader sequence, a short diversity sequence, a short joining sequence, a constant sequence and termination codons; in comparison, the light chain gene arrangement includes information for the promotor sequence, separate and termination codons. The transcript m RNA (i.e. messenger ribonucleic acid) of the immunoglobulin gene is comprised of a linear, continuous arrangement of complementary ribonucleic acid sequences. A recombinant expression vector encoding the expression of the continuous sequence could be prepared by reverse-transcription of the messenger ribonucleic acid into deoxyribonucleic acid. The expression vector is designed with selective markers and to afford expression of both heavy chains and light chains within a HCAb. Further, the genetic information is expressed by introducing the genes encoding immunoglobulin heavy and light chain synthesis into a vehicle suitable for expression such as bacteria, fungi, insect or mammmalian cell culture.

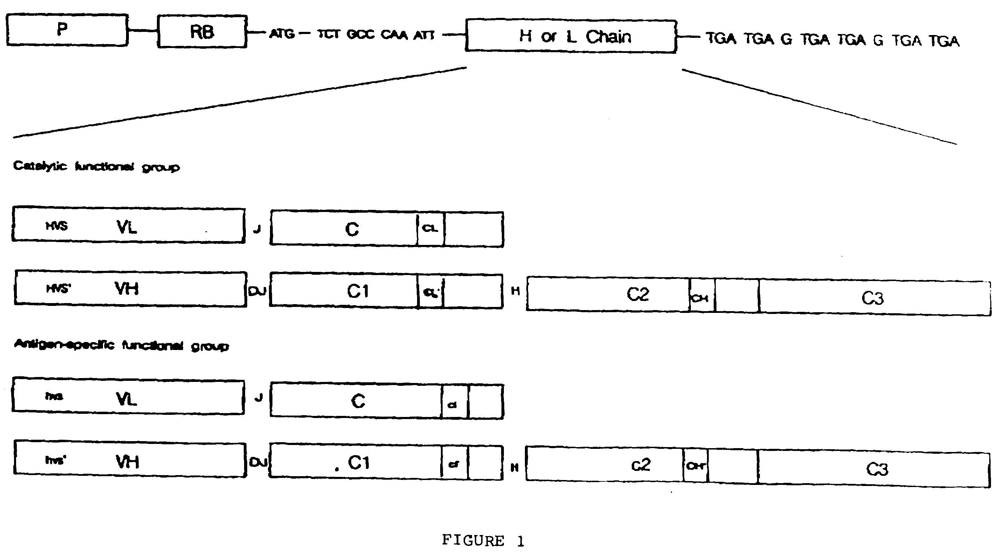

We have devised a recombinant scheme, and provide a general description hereof. Briefly, the expression system, illustrated in Figure 1, desirably includes a sequence of nucleotide bases for regulation of gene expression. The phosphoglycerate promotor sequence has been used for regulation of synthesis of complex molecules, including immunoglobulin, and is known to those practiced in the art. Said promotor (P) sequence is followed by a ribosome binding site (RB). A sequence containing a start codon (vv.ATG) and a leader sequence would also be constructed into the gene; the sequence TCT GCC CAA ATT is known to those practiced in the art of gene expression in yeast as a leader sequence which can afford secretion of the polypeptide. The leader sequence is immediately followed by sequences encoding the variable (V-) and constant domains (C-), respectively. Included in this arrangement would be the proximal orientation of information encoding the variable domain.

It is well known that hypervariable genetic code sequences exist within the variable domains of both heavy and light chain encoding gene sequences. Further, said hypervariable sequences encode complementary peptide sequences or complementary determining regions (vv. CDR) within the antigens (hvs or hvs') or catalytic (HVS or HVS') binding pocket of the HCAb immunoglobulin molecule. Regions containing said hypervariable sequences are flanked on either end by defined restriction enzyme cutting sites. The value of such a development is that it would permit removal, and replacement of CDR's in blocks, or "cassettes"; the latter term is commonly employed by those skilled in the art to refer to said moveable blocks of genetic information. This design thus allows innumerable alterations of catalytic or antigen specific sequences, and even introduction of sequences from biologically active molecules such as enzymes.

The variable domain is joined to the constant (C) domain of the light chain, or the first constant (C1) domain of the heavy chain, via short elements of joining (J or DJ) sequence code in a manner affording expression of protein structure consistent with the known organization of the immunoglobulin. Some of said sequences may be decreased or increased in accordance with design needs.

The pairing of immunoglobulin chains within the cell can be random with heavy chains and light chains pairing without consideration of the respective antigen or catalytic specificities. Such misannealing can reduce the efficient recovery of functionally active HCAbs. As a further development, the genes encoding the first constant domains of the heavy chains (CL' or cl') and the constant domain of the light chains (CL or cl) are modified to provide selectively complementary sequences between corresponding heavy-light chain pairs.

A sequence encoding a hinge (H) region is included, followed by all or part of the second constant domain (C2) of the heavy chain. As with light chain-heavy chain pairing, selectively complementary sequences would be similarly introduced within the code for heavy chain synthesis so as to only permit pairing of the catalytic (CH) antibody half with antigen-specific (CH') antibody half. Said sequence may then be followed by genetic information encoding the third constant region (C3) followed by a termination sequence.

Production of HCAbs of this invention is not limited to in vivo expression systems. Biochemical methods are also used. One of them used to produce an HCAb of the invention involved limited proteolysis and reduction of two distinct antibodies, one monoclonal antibody being a catalytic antibody such as MOPC 167 (readily otainable from the National Cancer Institute of the National Institutes of Health, Bethesda, MD., USA) and the other being any monoclonal antibody having specific binding affinity for an analyte of interest, to produce unifunctional F(ab') fragments, followed by reannealing and reoxidation to form interchain disulfide bonds. To favor the formation of HCAbs, the thiols of one antibody F(ab') are activated with Ellman's reagent or with the bifunctional crosslinking reagent O-phenylenedimaleimide before mixing with an equimolar amount of the second antibody F(ab') containing free thiols.

HCAbs of the invention obtained using any of the methods described above may be purified by affinity chromatography. Primary purification employs immobilized transition state analogue, with a secondary purification on an immobilized antigen column.

Purified HCAb may then be used for any diagnostic application, an immunoassay for analyte detection, requiring an enzyme-conjugated antibody such as among others, sandwich assay formats, and the current invention is not limited to any specific formats or protocols. Also, catalytic sites from existing enzymes such as alkaline phosphatase may be substituted for the complementary determining regions within the catalytic antibody molecules.

"Solid phase support" as used herein refers to an insoluble material to which one component of the assay may be bound. Known materials of this type include hydrocarbon polymers such as polystyrene and polypropylene, glass, metals, and gels. Such supports may be in the form of beads, tubes, strips, disks and the like. Magnetic particles are particularly preferred for use with the assays of this invention.

Such antibodies may also be useful as therapeutic compositions and in methods for treating diseases comprising target cells having antigenic determinants on the surface, the HCAb catalytic moiety providing the therapeutic activity. Such diseases, include cancers, non-malignant tumors and the like, when the antibody is toxic to the diseased target cells.

The invention may be better understood by reference to the following non-limiting examples.

### Example 1- Heterodifunctional catalytic antibody (HCAb) usable for ester specific hydrolysis and specific for humain IgG

### Preparation of hybridoma producing catalytic antibodies

Eight week old female BALB/c mice are immunized on a biweekly basis with p-aminophenylmethylphosphonate linked to tetanus toxoid via hemisuccimide in the para-position in Incomplete Freund's adjuvant by the intraperitoneal route. The mice are sacrificed by cervical dislocation after 10-20 weeks and the spleens surgically removed.
The spleens are transferred to a petri dish containing 5 ml of Dulbecco's modified medium (DME) containing 100 µg/ml gentamycin. The splenocytes are freed by tearing the tissue apart. The splenocytes are washed once with DME, by centrifugation at 250 g 5 minutes at 18-15°C and are then combined in a 5:1 ratio with washed SP2/O cells, a plasmocytoma cell line that can be obtained commercially.

The cells are pelleted, by centrifugation at 200 g for 6 minutes at 18-25°C, excess supernatant removed, and the cells are resuspended in 1 ml of buffered 50% polyethylene glycol solution. The cells are incubated at room temperature for 120 seconds prior to centrifugation at approximately 400 x g for 6 minutes. Cells are then centifuged, at 250 g for 5 minutes at 18-25°C, gently rinsed with DME, resuspended in Iscove's Modified DME with SP2/O conditioned medium and gently transferred to 75 cm³ flask.

Following an overnight incubation at 37°C in 5% CO₂, in air, the cells are diluted to 2x10⁶ cells/ml in conditioned medium, and seeded into 96 well plates (250 µl/well). After 7-10 days, the supernatants are screened as follows. 96 well flat-bottom polystyrene plates are presensitized overnight with 100mg/well goat anti-mousse IgG. The plates are washed, and then blocked with 1% bovine serum albumin (BSA) in 50 mM phosphate buffered saline, pH 7.2 for 30 minutes, washed and dried out. A 100 µl aliguot supernatant is added to each well.

The substrate, p-nitrophenyl acetate, is diluted into a neutral or phosphate-free buffer (15 m M Tris, 15mM NaCl, pH7.0) added to each well and incubated for up to 24 hours. Plates are periodically examined for signs of ester hydrolysis signified by the appearance of free p-nitrophenol, yellow chromogen. Positive clones are expanded and evaluated for evidence of hydrolytic activity with a luminogenic luminescent substrate, such as AMPPD and luminescent signal is measured.

### Preparation of Fusion Partner

Murine hybridomas secreting monoclonal antibodies with antigen specificity toward human IgG are subjected to levels of selection to obtain a suitable fusion partner. First, cells lacking hypoxanthine phosphoribosyl transferase are identified as follows. 10⁸ exponentially growing cells in Iscove's DMWM with 20 % fetal bovine serum are harvested, washed, and resuspended in medium containing 6.6 m M 8-azaguanine. Cells are grown in the presence of 8-azaguanine for about 24 hours or until approximately 80 % of the cells have lost viability. The cells are then washed, and resuspended in the above culture medium devoid of 8-azaguanine, and cultured until they reach a mass of approximately 10⁸ cells as determined by cell counts. This process is repeated two more times. The cells are then subjected to a final selection in 8-azaguanine by culture for 5 days in the presence of the inhibitor.

Verification of the HGPRT⁻ defect is confirmed by culture in HAT containing Iscove's medium. The cells are the reselected for resistance to ouabain. The selection process is the same are that described above for 8-azaguanine. Cells expressing antibody to IgG, and which are HGPRT⁻ and ouabain resistant are selected.

### Fusion of Hybridomas

The hybridomas obtained according to the processes previously described are fused by combining them in a 1:5 hybridoma to target cell ratio, in 1 ml of buffered 50 % polyethylene glycol solution. The cells are incubated at room temperature for 120 seconds prior to centrifugation at approximately 400 x g for 6 minutes. All cells are centrifuged, gently rinsed with DME, resuspended in Iscove's Modified DME with SP2/0 conditioned medium and gently transferred to 75 cm² flask.

Following an overnight incubation at 37°C in 5 % CO₂, the cells are diluted to 2x10⁶ cells/ml in conditioned medium, and seeded into 96 well plates (250 µl/well). After 7-10 days, the supernatants are screened using 96 well flat-bottom polystyrene plates presensitized overnight with human IgG. The plates are washed, and then blocked with 1 % bovine serum albumin (BSA) in 50 mM phosphate buffered saline, pH 7.2 for 30 minutes, washed and dried out. A 100 µl aliquot of supernatant is added to each well and the substrate, p-nitrophenyl acetate, diluted into a neutral or slightly acidic phosphate-free buffer before incubation for up to 24 hours. Plates are periodically examined for signs of ester hydrolysis signified by the appearance of free p-nitrophenol, yellow chromogen. Positive clones are expanded as above and evaluated for evidence of hydrolytic activity with a luminescent substrate. Wells exhibiting substrate conversion are identified as containing hybrid clones able to both recognize the antigen (i.e. human IgG, in the instance), and cleave the separate substrate.

### Example 2 HCAb with phosphorylcholine esterase activity and affinity for human IgG.

MOPC 167, a widely available clone with a described phosphorylcholine carbonate cleaving activity, described by Schultz et al. in Science 234 1570 (1986), is fused with hybridomas producing antibodies specific for human IgG's, resulting in a quadroma.

The IgG specific clone is back-selected for combined 8-azaguanine resistance and ouabain sensitivity according to the methods described in Example 1. Cell fusion is carried out as described previously. Following plating and several days growth in culture, the cell supernatants are screened using 96 well plates sensitized with purified human IgG. Culture supernatant is transferred to the plates, incubated for a period of time, and washed with a buffered solution.
S-Acetylthiocholine (i.e the substrate), which can be cleaved by the catalytic antibody produced by MOPC 167 is added to the plates. Wells exhibiting substrate conversion are identified as containing HCAb.

### Example 3 HCAb with phosphorylcholine esterase activity and affinity for theophylline :

Clone 9-49 is a hybridoma producing monoclonal antibody to theophylline and is available from Sanofi Diagnostics Pasteur (Chaska, MN 55318, USA).
Any theophylline-specific monoclonal antibody can be used instead of Mab 9-49 in this example.
Hybridoma clone 9-49 cells are fused with clone MOPC 167 cells. Cell fusion is carried out as described previously.

Selection of fused cells, characterization of the catalytic activity and theophylline buiding capacity of the newly formed HCAb (i.e. MOPC 167 x 9-49) is performed exactly as in Example 2, except that theophylline presensitized microplates (or others solid support) are used instead of goat-anti mouse IgG presensitized plates.

### Example 4 Biochemical Preparation of HCAb with phosphorylcholine esterase activity and affinity theophylline.

Preparation of ANTIBODIES. Both MOPC 167 and 9-49 were propagated in primed BALB/c mice.

MOPC 167 antibody was isolated from ascitic fluid by immunoaffinity chromatography on Affigel ®G10 with bound p-aminophenyl phosphorylcholine and eluted with Pierce gentle elution buffer.

Separately, 9-49 monoclonal antibody was isolated by successive fractionation using a DE-S2 Whatman ® column and 0.3 M sodium phosphate buffer, pH 7.2, followed by size separation over a Sephacryl ® G-200 column equilibrated with 0.15 M PBS, pH 7.4.

PREPARATION OF FAB'S. Each of the aforementioned antibody preparations was dialyzed overnight into 100 mM sodium acetate buffer, pH 4.3 at 4° C. Then in order to determine the best enzymic digestion process, each antibody was contacted with hog stomach pepsin in a 100:1 mass ratio at 37°C, samples were drawn at several timepoints, and the digestion products analyzed by SDS-PAGE gel electrophoresis using a Pharmacia® PHAST system. The optimal digestion time for MOPC 167 was 4 hours and 2 hours for 9-49.
The antibody preparations were digested according to the observed incubation time optima, and the reaction stopped by raising the pH to 8. Digestion products were then separated into 200 mM Tris-HC1, pH 8.0 containing 10 mM EDTA, via a Biocryl ® matrix on a Pharmacia ® FPLC system with a flow rate of 1 ml/minute. The F(ab')2 fraction of each antibody so obtained was further reduced by addition of 2-mercaptoethanol to a final concentration of 20 mM, and incubated for 30 minutes at 37°C; all subsequent reaction steps were carried out at 4°C. Each resulting Fab' was separated from the 2-mercaptoethanol in excess by passage over a Sephadex® G-25 column equilibrated with 50 mM sodium acetate pH 5.3, with 0.5 mM EDTA.

Two volumes of 9-49 Fab' fraction were slowly combined with one volume of 12 mM o-phenylenedimaleimide dissolved in anhydrous dimethylformamide. After approximately 30 minutes, the reactants were passaged through a Sephadex ® G-25 column equilibrated with 50 mM sodium acetate pH 5.3, with 0.5 mM EDTA.

### PREPARATION OF MOPC 167 x 9-49 HCAb

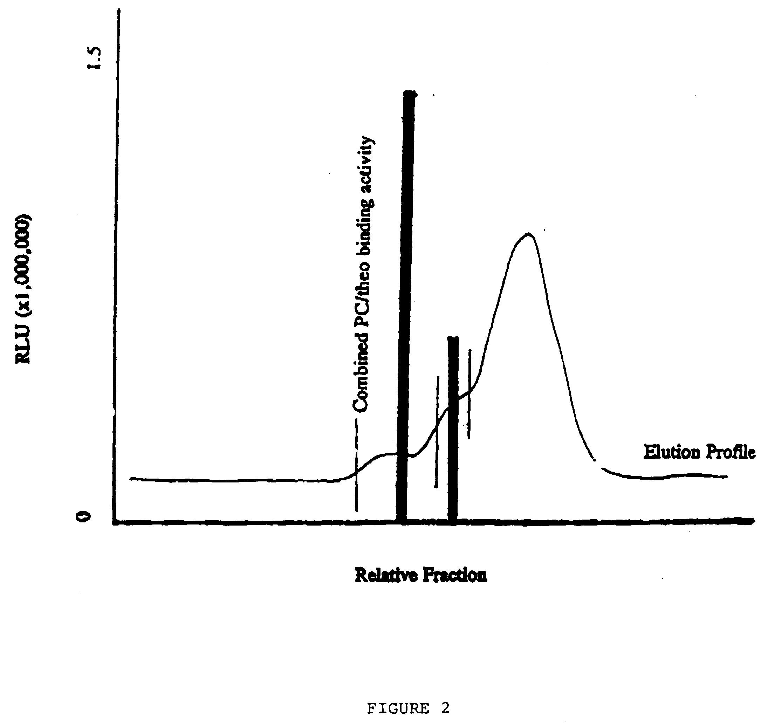

The modified 9-49 Fab' was combined with MOPC 167 Fab' in a 5:1 ratio, concentrated to approximately 5 mg/ml total protein concentration, and incubated overnight at about 4-5°C. The resulting reactants were filtered through a 0.22 µm membrane, and separated by size exclusion chromatorgraphy through a Sephacryl ® column into Tris-HC1 buffer, pH 8.0 with EDTA. (See figure 2). The resulting bifunctional antibody eluted from the size exclusion column with an average apparent molecular weight of approximately 120 kD (the first vertical bar on Figure 2) as determined with molecular weight standards. This material was used in our subsequent studies.

### Example 5- Assays of HCAb

SUBSTRATE PREPARATION. p-nitrophenylcarbonylcholine was prepared from p-nitrophenylchloroformate and choline under anhydrous conditions according to the method of Shokat et al. in Ann. Rev. Immunol 8 335 (1990). The reaction product was exhaustively rinsed with dry pyridine. Following removal of solvent, the reaction product was observed to exhibit a melting temperature of 158°C, and decomposed to a yellow liquid at 160°C. The material was further submitted for analysis by mass and infrared spectroscopy.

ENZYMEIMMUNOASSAY FOR DETECTION OF THE BIFUNCTIONAL ANTIBODY. MOPC 167, 9-49 and MOPC 167 x 9-49 antibody fractions, and normal mouse IgG wera separately diluted to 10 µg/ml in 150 mM Tris, pH 8.0 with 1 % bovine serum albumin. One ml aliquote of each diluted antibody was combined with 1 mg of washed magnetic particles (Rhone Poulenc) sensitized with p-aminophenylphosphorylcholine. After 1 hr., the particles were washed with the binding buffer, and combined with 1 µg of alkaline phosphatase labelled with theophylline (1:3 ratio). Following 15 minutes incubation at room temperature, the particles were washed with 100 mM Tris, (pH 8.0) with 0.05 % FC-100 a surfactant available from 3M Corporation, St Paul, USA. Lumiphos ® available from Lumigen (USA) was added to each tube, and signal measured on a Berthold luminometer.

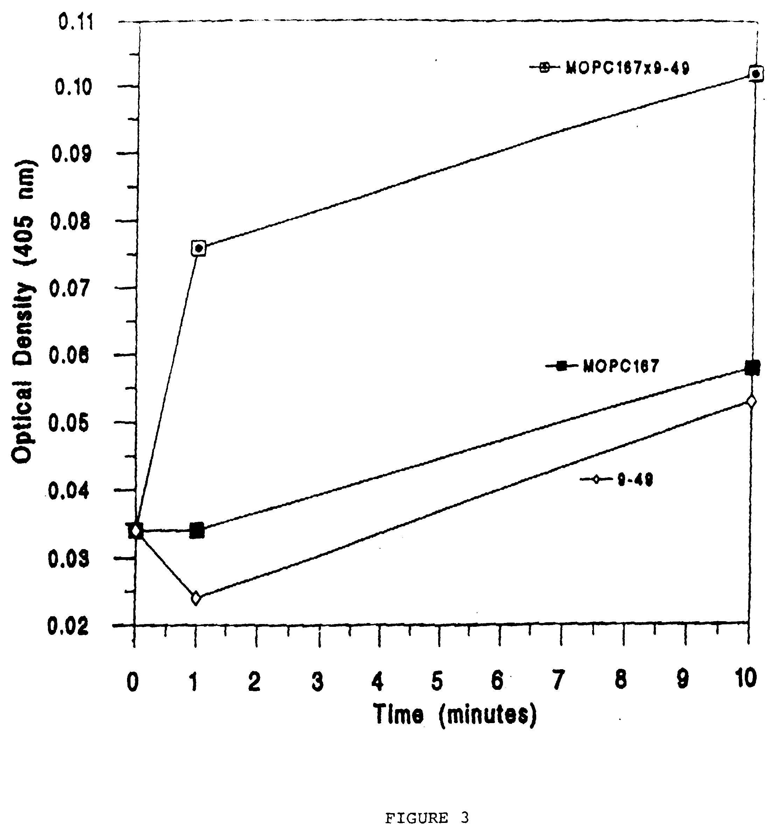

IMMUNOASSAY BASED DETECTION OF CATALYTIC CONVERSION OF P-NITROPHENYLCARBONYLCHOLINE. 100 µg of MOPC 167, 9-49, MOPC 167 x 9-49 or normal murine IgG was separately mixed with 1 mg of either theophylline sensitized or goat immunoglobulin coated magnetic particles for 2 h. The particles were then washed with MOPC buffer (15 mM Tris, pH 7.0 with 15 mM sodium chloride). p-nitrophenylcarbonylcholine was dissolved to 25 mM in MOPC buffer. The substrate solution was added to the particules mixed by vortex, and incubated at 29.3°C until measurement (see Fig 3); at 30 seconds prior to each reading, the particles were magnetically separated, and the absorbance read at 410 nm on a Hewlett Packard® diode array spectrophotometer.

Fractions inclusive of the major portion containing the 120 kD molecule and lower molecular weights were tested for the capacity to concurrently bind theophylline and a choline derivative by the immunoassay procedures described above. The results indicated that the fractions averaging a molecular weight of 120 kD were able to retain 85-100 % of an enzyme-labelled theophylline conjugate on choline derivative sensitized particles, whereas the next lower molecular weight pool examined retained only one-third of the total added conjugate activity. This observation would be expected to reflect the dilution of intact bifunctional antibody, and an increase in monofunctional Fab'.

In another experiment the bifunctional antibody molecule was bound in the reverse orientation with the theophylline derivative affixed to the solid phase. Figure 3 illustrates the increased rate of substrate conversion associated with the system containing bifunctional antibody, and no discernible increase with monofunctional Fab's included in the assay. No increase of substrate conversion was observed when solid-phase lacking the theophylline derivative was employed (not shown); this observation was construed to support the contention that both antigen-targeting and substrate conversion activities must be associated within the same molecule in this type of assay.

Thus, our antibody product having a molecular weight of about 120 kD showed dual recognition of theophylline and choline derivatives (Figure 2) and the catalytic conversion of p-nitrophenylcarbonylcholine when it is concomitantly associated with theophylline immobilized on particles (Figure 3). This coupling was unusual in that the parent antibodies were of the A(K) and G1(k) isotype, respectively.

Competitive Assay for an Antigen.

In setting the assay, 200 µl of antigen containing samples or standards in 10 mM Tris containing 5 µM KCN, pH 8.0 are transferred to 10x60 mm borosilicate glass test tubes. To each reaction tube is added 100 µl (0.1 µg) of the HCAb which specifically binds the antigen and which catalyzes a reaction with a particular substrate. The reactants are incubated for 15 minutes at 37°C in a shaker water bath agitating at approximately 60 rpm. 50 µl of particles are added to the reaction mixture, and incubated at 37°C with agitation for an additional 30 minute time span. The reaction is stopped by sedimentation for 2 minutes in a magnetic field. The tubes are rinsed twice with 1 ml of 50 mM Tris, 0.15 M NaCl, 0.1 % Triton® X-100, pH 7.4. 200 µl of substrate is added to each tube, and the reaction initiated by static incubation at 37°C for 5 minutes. The tubes are then cooled to room temperature for 2 minutes, and the resulting signal detected using a spetrophotometer or a luminometer, depending on the substrate used.

### Example 6 - Preparation of a Therapeutic Composition

HCAbs of this invention can be used in the treatment of certain diseases with cell-bound targets. A HCAb can be obtained using the above described methods, wherein part of the antibody molecule recognize a unique cellular marker, i.e., carcinoembryonic antigen (CEA) and part of the antibody molecule has a toxic activity disphosphoribo-transferase-NAD-transferase (diptheria toxin) or activates a potentially toxic compound.

The therapeutic composition is injected in a patient having the disease and the antibody molecules bind to cells bearing the marker. Pending internalization of the antibody, the catalytic half of the molecule specifically transfers nicotine-adeninedinucleotide to elongation factor 2, whereupon certain critical anabolic processes cease to function resulting in target cell death.

## Claims

1. A heterobifunctional catalytic antibody useful in a diagnostic assay, in the manner of an enzyme-labelled antibody, said antibody comprising a constant region and two Fab fragments, the variable domain of the first Fab fragment exhibiting intrinsic catalytic activity for a substrate and the variable domain of the second Fab fragment having antigen binding specificity for an antigen, wherein said antigen is different from said substrate, in such a way that the catalytic activity for said substrate can be correlated with the binding of said antigen.

2. The heterobifunctional catalytic antibody of claim 1 wherein the constant region consists of two constant domains joined respectively to each of the variable domains.

3. The heterobifunctional catalytic antibody of claim 1 wherein the catalytic activity of the antibody is that of a phosphatase.

4. The heterobifunctional catalytic antibody of claim 1 or 2 wherein the catalytic part of the antibody is derived from the MOPC 167 antibody.

5. A method for preparing a heterobifunctional catalytic antibody according to claims 1 to 4, comprising :
a) fusing hybridomas producing monoclonal catalytic antibodies with either lymphocytes or hybridomes producing antibodies against the desired antigen or hapten,
b) culturing the hybrid cells obtained in a)
c) selecting the clones producing antibodies directed against said antigen or hapten and having the catalytic activity.

6. A method for preparing a heterobifunctional catalytic antibody according to claims 1 to 4, comprising :
a) transfecting into a cell or microorganism, a recombinant expression vector, the DNA of which encodes the antibody heavy and light chains such as the first variable region of the antibody has the desired antigen or hapten specificity and the second variable region has the desired catalytic property,
b) growing the cells or microorganisms in such a way that said antibody is expressed,
c) collecting the antibody.

7. A method according to claim 6 wherein the DNA region coding for the constant region of the antibody comprises a sequence which forces the subsequent annealing of light chain/heavy chain and heavy chain/heavy chain necessary combinations.

8. A method for preparing a heterobifunctional catalytic antibody according to claims 1 to 4, comprising :
a) reducing the disulfide bonds linking the two heavy chains of a catalytic antibody and those of an antigen binding antibody,
b) combining the two types of resulting chains and reacting them with a crosslinking agent capable of reassembling heavy chains of antibodies, and
c) recovering reassembled antibodies possessing catalytic properties together with affinity for the antigen.

9. A method for assaying for an analyte in a sample comprising ;
a) contacting the sample with a known amount of a heterobifunctional catalytic antibody according to claim 1 to allow the antibody to bind analyte present in the sample,
b) separating bound antibody from unbound antibody ;
c) measuring the amount of catalytic activity associated with either the analyte-antibody complex or the amount of unbound antibody by mixing them with the substrate to determine the amount of analyte present.

10. A kit for immunoassay comprising :
- a heterobifunctional catalytic antibody according to claims 1 to 4,
- a substrate of the catalytic antibody,
- a means for detecting the transformation of the substrate.

## Patentansprüche

1. Heterobifunktioneller, katalytischer Antikörper, der in einem Diagnoseassay in der Weise eines enzym-markierten Antikörpers verwendbar ist, wobei der Antikörper eine konstante Region und zwei Fab-Fragmente umfaßt, wobei die variable Domäne des ersten Fab-Fragments eine innere katalytische Aktivität für ein Substrat zeigt und die variable Domäne des zweiten Fab-Fragments Antigenbindungsspezifizität für ein Antigen besitzt, wobei das Antigen von dem Substrat verschieden ist, derart, daß die katalytische Aktivität für das Substrat mit der Bindung des Antigens korreliert werden kann.

2. Heterobifunktioneller, katalytischer Antikörper nach Anspruch 1, wobei die konstante Region aus zwei konstanten Domänen besteht, die respektive mit jeder der variablen Domänen verbunden sind.

3. Heterobifunktioneller, katalytischer Antikörper nach Anspruch 1, wobei die katalytische Aktivität des Antikörpers diejenige einer Phosphatase ist.

4. Heterobifunktioneller, katalytischer Antikörper nach Anspruch 1 oder 2, wobei der katalytische Teil des Antikörpers von dem MOPC 167-Antikörper abgeleitet ist.

5. Verfahren zur Herstellung eines heterobifunktionellen, katalytischen Antikörpers gemäß den Ansprüchen 1 bis 4, umfassend:
a) Fusionieren von Hybridomas, welche monoklonale, katalytische Antikörper produzieren mit entweder Lymphozyten oder Hybridomen, welche Antikörper gegenüber dem erwünschten Antigen oder Hapten produzieren,
b) Kultivieren der in a) erhaltenen Hybridzellen,
c) Auswählen der Klone, welche gegen das Antigen oder Hapten gerichtete Antikörper produzieren und die katalytische Aktivität aufweisen.

6. Verfahren zur Herstellung eines heterobifunktionellen, katalytischen Antikörpers gemäß den Ansprüchen 1 bis 4, umfassend
a) Transfektion in eine Zelle oder Mikroorganismus eines rekombinanten Expressionsvektors, dessen DNA die schweren und leichten Ketten des Antikörpers encodiert, so daß die erste variable Region des Antikörpers die erwünschte Antigen- oder Haptenspezifizität und die zweite variable Region die erwünschte katalytische Eigenschaft besitzt,
b) Wachsenlassen der Zellen oder Mikroorganismen in der Weise, daß der Antikörper exprimiert wird,
c) Sammeln des Antikörpers.

7. Verfahren nach Anspruch 6, wobei die DNA-Region, welche für die konstante Region des Antikörpers codiert, eine Sequenz umfaßt, welche die nachfolgende Anelierung der notwenigen Kombinationen aus leichte Kette/schwere Kette und schwere Kette/schwere Kette erzwingt.

8. Verfahren zur Herstellung eines heterobifunktionellen, katalytischen Antikörpers gemäß den Ansprüchen 1 bis 4, umfassend
a) Reduzieren der Disulfidbindungen, welche die zwei schweren Ketten eines katalytischen Antikörpers und diejenigen eines antigenbindenden Antikörpers verknüpfen,
b) Kombinieren der zwei Typen der resultierenden Ketten und Umsetzen dieser mit einem Vernetzungsmittel, welches fähig ist, schwere Ketten von Antikörpern neu anzuordnen, und
c) Gewinnen neu angeordneter Antikörper, welche katalytische Eigenschaften gepaart mit einer Affinität für das Antigen besitzen.

9. Verfahren zur Prüfung auf einen Analyt in einer Probe, umfassend:
a) Kontaktieren der Probe mit einer bekannten Menge eines heterobifunktionellen, katalytischen Antikörpers gemäß Anspruch 1, um den Antikörper den in der Probe vorliegenden Analyt binden zu lassen;
b) Abtrennen des gebundenen Antikörpers von ungebundenem Antikörper;
c) Messen des Ausmaßes der katalytischen Aktivität, welche mit entweder dem Analyt-Antikörper-Komplex oder der Menge an ungebundenem Antikörper assoziiert ist, durch Vermischen dieser mit dem Substrat, um die Menge des vorliegenden Analyts zu bestimmen.

10. Kit für ein Immunoassay, umfassend:
- einen heterobifunktionellen, katalytischen Antikörper gemäß den Ansprüchen 1-4,
- ein Substrat des katalytischen Antikörpers,
- ein Mittel beziehungsweise eine Einrichtung zur Bestimmung der Transformation des Substrats.

## Revendications

1. Anticorps hétérobifonctionnel catalytique utile dans un test de diagnostic, à la manière d'un anticorps marqué avec une enzyme, ledit anticorps comprenant une région constante et deux fragments Fab, le domaine variable du premier fragment Fab présentant une activité catalytique intrinsèque pour un substrat et le domaine variable du second fragment Fab ayant une spécificité de liaison antigènique pour un antigène, où ledit antigène est différent dudit substrat, de telle sorte qu'une corrélation peut être établie entre l'activité catalytique pour ledit substrat et la liaison dudit antigène.

2. Anticorps hétérobifonctionnel catalytique selon la revendication 1, dans lequel la région constante est constituée de deux domaines constants respectivement liés à chacun des domaines variables.

3. Anticorps hétérobifonctionnel catalytique selon la revendication 1, dans lequel l'activité catalytique de l'anticorps est celle d'une phosphatase.

4. Anticorps hétérobifonctionnel catalytique selon la revendication 1 ou 2, dans lequel la partie catalytique de l'anticorps est dérivée de l'anticorps MOPC 167.

5. Procédé de préparation d'un anticorps hétérobifonctionnel catalytique selon les revendications 1 à 4, comprenant:
a) la fusion d'hybridomes produisant des anticorps monoclonaux catalytiques, soit avec des lymphocytes, soit avec des hybridomes produisant des anticorps dirigés sontre l'antigène ou l'haptène désiré.
b) la culture des cellules hybrides obtenues en a)
c) la sélection des clones qui produisent des anticorps dirigés contre ledit antigène ou haptène et possédant l'activité catalytique.

6. Procédé de préparation d'un anticorps hétérobifonctionnel catalytique selon les revendications 1 à 4, comprenant:
a) la transfection dans une cellule ou microorganisme d'un vecteur d'expression recombinant, dont l'ADN code pour les chaînes légères et lourdes de l'anticorps de façon à ce que la première région variable de l'anticorps possède la spécificité désirée vis-à-vis de l'antigène ou de l'haptène et la seconde région variable, possède la propriété catalytique désirée
b) la culture des cellules ou microorganisme dans des conditions telles que ledit anticorps soit exprimé,
c) la récupération de l'anticorps.

7. Procédé selon la revendication 6 dans lequel la région d'ADN codant pour la région constante de l'anticorps contient une séquence qui entraine l'hybridation ultérieure des chaîne légère/chaîne lourde et les combinaisons nécessaires chaîne lourde/chaîne lourde.

8. Procédé de préparation d'un anticorps hétérobifonctionnel catalytique selon les revendications 1 à 4, comprenant:
a) la réduction des ponts disulfures qui lient les deux chaînes lourdes d'un anticorps catalytique et celles d'un anticorps qui se lie à un antigène,
b) la combinaison des deux types de chaînes résultantes et la réaction de ces chaînes avec un agent de réticulation capable de réassocier les chaînes lourdes des anticorps et
c) la récupération des anticorps réassociés possédant des propriétés catalytiques ainsi qu'une affinité pour l'antigène.

9. Procédé de détection d'un produit à analyser dans un échantillon comprenant:
a) la mise en contact de l'échantillon avec une quantité connue d'un anticorps hétérobifonctionnel catalytique selon la revendication 1 de manière à permettre à l'anticorps de se lier au produit à analyser présent dans l'échantillon,
b) la séparation de l'anticorps lié de l'anticorps non lié,
c) le dosage de l'activité catalytique associée soit au complexe produit à analyser-anticorps soit à l'anticorps non lié en les mélangeant avec le substrat afin de déterminer la quantité de produit à analyser présent.

10. Kit de test immunologique comprenant:
- un anticorps hétérobifonctionnel catalytique selon les revendications 1 à 4,
- un substrat de l'anticorps catalytique,
- un procédé de détection de la transformation du substrat.
